# EUROPÄISCHE PATENTSCHRIFT

(11) **EP 1 990 002 B1**
(45) Veröffentlichungstag und Bekanntmachung des Hinweises auf die Patenterteilung: **16.12.2009**
(21) Anmeldenummer: 07009456.0
(22) Anmeldetag: 11.05.2007
(51) Int. Cl.: A61B 5/15, G01N 35/00

(54) **Stechsystem, Lanzettenvorratssystem, Verfahren zur Herstellung eines solchen Lanzettenvorratssystem und Verfahren zum Positionieren von auf einem Trägerband angeordneten Funktionselementen**
Pricking system, lancet storage system, method for manufacturing such a lancet storage system and method for positioning functional elements arranged on a carrier belt
Système de connexion, système de réserve de lancettes, procédé destiné à la fabrication d'un tel système de réserve de lancettes et procédé destiné au positionnement d'éléments fonctionnels disposés sur une bande de transport

(43) Veröffentlichungstag der Anmeldung: 12.11.2008
(73) Patentinhaber: Roche Diagnostics GmbH, 68305 Mannheim (DE); F. Hoffmann-La Roche AG, 4070 Basel (CH)
(72) Erfinder: Harttig, Herbert, 67434 Neustadt (DE); Hiller, Bernd, 68623 Lampertheim (DE); Konya, Ahmet, 67165 Waldsee (DE); Kube, Oliver, 67547 Worm (DE); Kuhr, Hans-Jürgen, 68219 Mannheim (DE); Wehowski, Frederic, 68766 Hockenheim (DE)
(74) Vertreter: Twelmeier Mommer & Partner

(56) Entgegenhaltungen:
- EP-A- 0 371 572
- EP-A- 1 739 432
- WO-A-00/15653
- WO-A-2004/057345
- WO-A-2004/060174
- WO-A-2005/104948
- US-A1- 2005 245 845

## Beschreibung

Die Erfindung geht aus von einem Stechsystem mit den Im Oberbegriff des Anspruchs 1 angegebenen Merkmalen, wie es aus der WO 20041057345 A2. bekannt ist.

Aus der WO 00/15653 A2 ist ein Archivband für chemische Substanzen bekannt, auf dem Abstände einzelnen Proben zu einer Markierung oder dem Bandanfang codiert sind, so dass die Positionen dieser Proben leichter angefahren werden können.

Zu einem derartigen Stechsystem gehört als Lanzettenvorrat ein Trägerband, das mehrere Lanzetten trägt. Diese Lanzetten werden durch Bewegen des Trägerbandes in einer Förderrichtung nacheinander in eine Gebrauchsposition gebracht, in der sie mit einem Stechantrieb für eine Stechbewegung beschleunigt werden können, so dass in einem an eine Gehäuseöffnung des Stechsystems angelegten Körperteil eine Stichwunde zur Gewinnung einer Körperflüssigkeitsprobe erzeugt werden kann.

Derartige Stechsysteme werden beispielsweise von Diabetikern verwendet, die mehrmals täglich ihren Blutzuckerspiegel überprüfen müssen und dafür eine Körperflüssigkeitsprobe, in der Regel Blut oder Interstitielle Flüssigkeit benötigen, die aus einer mit einem Stechsystem erzeugten Stichwunde gewonnen wird.

Im Gegensatz zu Stechsystem mit Trommelmagazinen, die typischerweise nur sechs oder acht Lanzetten enthalten, kann ein Trägerband einen Lanzettenvorrat mit einer wesentlich größeren Anzahl von Lanzetten bilden. Stechsysteme mit einem Lanzettenvorrat in Form eines Trägerbands haben deshalb den Vorteil, dass sich ein Benutzer seltener um den Austausch eines Trägerbandes oder, bei Verwendung von Wegwerfgeräten, um ein neues Gerät bemühen muss.

Damit mit einer Lanzette eines Trägerbandes in ein an eine Gehäuseöffnung angelegtes Körperteil eingestochen werden kann, muss die Lanzette in einer Gebrauchsposition in Bezug auf die Gehäuseöffnung positioniert werden. Die dabei zulässigen Positionierungenauigkeiten sind recht klein, so dass insbesondere bei einem sehr langen Trägerband ein erheblicher Aufwand erforderlich ist.

Aufgabe der Erfindung ist es deshalb, einen Weg aufzuzeigen, wie bei einem Stechsystem mit geringerem Aufwand eine präzise Positionierung der Lanzetten eines Trägerbandes in einer Gebrauchsposition erreicht werden kann.

Diese Aufgabe wird mit einem Stechsystem mit den im Anspruch 1 angegebenen Merkmalen gelöst. Die Aufgabe wird ferner durch ein Verfahren zur Herstellung eines Lanzettenvorratssystems mit den im Anspruch 13 angegebenen Merkmalen sowie ein Verfahren zum Positionieren von auf einem Trägerband angeordneten Funktionselementen, insbesondere Lanzetten, in einer Gebrauchsposition mit den im Anspruch 14 angegebenen Merkmalen gelöst. Vorteilhafte Weiterbildungen der Erfindung sind Gegenstand der abhängigen Unteransprüche.

Um auf einem Trägerband angeordnete Testfelder, mit denen eine Körperflüssigkeitsprobe untersucht werden kann, präzise in eine Gebrauchsposition zu positionieren, ist es aus der EP 1 739 432 A1 bekannt, das Trägerband mit Wegmarkierungen zu versehen, die beim Bandtransport eine Wegerfassung ermöglichen. Durch eine präzise Anordnung der Testfelder in Bezug auf die Wegmarkierungen ist auf diese Weise eine präzise Positionierung der Testfelder in einer Gebrauchsposition möglich.

Erfindungsgemäß werden auf einem Trägerband angeordnete Lanzetten oder andere Funktionselemente, beispielsweise Testfelder, mittels auf dem Trägerband angebrachter Positionsmarken in einer Gebrauchsposition positioniert. Dabei ist es jedoch weder erforderlich, dass die Positionsmarken mit hoher Präzision äquidistant auf dem Trägerband angeordnet sind, noch ist es erforderlich, dass die Funktionselemente stets mit präzise gleichem Abstand in Bezug auf eine Positionsmarke angeordnet sind. Erfindungsgemäß können deshalb bei der Herstellung eines Trägerbandes erhebliche Kosten eingespart werden. Überraschenderweise ist trotz der unpräzisen Anordnung auf dem Trägerband eine hochpräzise Positionierung von Funktionselemente eines Trägerbandes in einer Gebrauchsposition möglich.

Erfindungsgemäß werden nämlich für die Funktionselemente auf einem Speichermedium Entfernungswerte gespeichert, die jeweils von der Entfernung des betreffenden Funktionselements des Trägerbandes von einer ihm zugeordneten Positionsmarke abhängen. Erfindungsgemäß wird mit einem Sensor eine Positionsmarke erfasst und daraufhin anhand des auf dem Speichermedium für die zu positionierende Lanzette gespeicherten Entfernungswerts bestimmt, wie weit das Trägerband mit dem Funktionselement nun bewegt werden muss, bis das Funktionselement die Gebrauchsposition erreicht. Bevorzugt wird das Trägerband dabei stets nur in Förderrichtung, also vorwärts, bewegt. Prinzipiell ist es aber auch möglich, dass das Trägerband nach dem Detektieren einer Positionsmarke um einen Positionierweg zurückbewegt wird, dessen Länge ebenso wie die Richtung durch den gespeicherten Entfernungswert bestimmt ist.

Die Entfernungswerte können jeweils den Abstand eines Funktionselements von einer Positionsmarke, insbesondere einer benachbarten Positionsmarke, angeben. Möglich ist es aber auch, dass die Positionsmarken Sollpositionen definieren, auf denen bei der Fertigung Lanzetten oder andere Funktionselemente abgelegt werden sollen, und die Entfernungswerte eine beim Herstellungsprozess aufgetretene Abweichung der tatsächlichen Position eines Funktionselementes von der Sollposition angeben. Beispielsweise kann vorgesehen sein, Funktionselemente bei der Fertigung eines Trägerbandes in einer Sollposition exakt zwischen benachbarten Positionsmarken anzuordnen. Anstatt anzugeben, wie weit ein Funktionselement von einer in Förderrichtung oder entgegen der Förderrichtung nächstliegenden Positionsmarke entfernt ist, kann ein Entfernungswert also beispielsweise auch angeben, wie weit das betreffende Funktionselement in einer gegebenen Richtung von einer Sollposition entfernt ist.

Möglich ist es auch, dass die Entfernungswerte beispielsweise die Länge eines Positionierwegs, also einer Strecke angeben, um die das Band zum Positionieren des Funktionselements in der Gebrauchsposition noch zu bewegen ist, wenn die betreffende Positionsmarke von dem Sensor des Stechsystems detektiert wird. Es ist deshalb nicht zwingend erforderlich, dass zur Positionierung eines Funktionselements aus einem gespeicherten Entfernungswert tatsächlich der Abstand des Funktionselements zu einer Positionsmarke berechnet wird. Es genügt, dass mit dem Entfernungswert bestimmt wird, wann das Trägerband gestoppt werden muss, um ein Funktionselement präzise in der Gebrauchsposition zu positionieren.

Zum Ermitteln der Entfernungswerte wird bevorzugt bei einem Trägerband, auf das Lanzetten oder andere Funktionselemente aufgebracht wurden, die exakte Position eines Funktionselements in Relation zu einer ihm zugeordneten Positionsmarke, bevorzugt der entgegen der Förderrichtung nächstliegenden, vermessen. Die Entfernungswerte können beispielsweise als Strichcode auf dem Trägerband selbst, beispielsweise auf dessen Rückseite, angebracht werden. Als Speichermedium können beispielsweise auch magnetische Speichermedien, Speicherchips, RFID-Label oder andere geeignete Datenträger verwendet werden. Das Speichermedium kann an dem Trägerband selbst oder einer Bandkassette, die bestimmungsgemäß in ein Stechgerät eingelegt wird, angebracht werden. Möglich ist es aber auch, das Speichermedium als separates Speicherelement vorzusehen.

Erfindungsgemäß können nicht nur Lanzetten sondern auch andere auf einem Trägerband positionierte Funktionselemente, beispielsweise Testfelder zur Untersuchung einer Körperflüssigkeitsprobe, präzise in einer Gebrauchsposition positioniert werden. Ein Aspekt der Erfindung betrifft deshalb ein Verfahren zum Positionieren von auf einem Trägerband angeordneten Funktionselementen in einer Gebrauchsposition durch Bewegen des Trägerbandes in Längsrichtung, dadurch gekennzeichnet, dass auf dem Trägerband angebrachte Positionsmarken mittels eines Sensors detektiert werden und daraufhin eine Vorwärtsbewegung des Trägerbandes um einen Positionierweg bewirkt wird, dessen Länge von einem gespeicherten Entfernungswert abhängt, der von der Entfernung des zu positionierenden Funktionselements von der detektierten. Positionsmarke abhängt.

Ein derartiges Verfahren wird bei einem erfindungsgemäßen Stecksystem angewendet, um Lanzetten in der Gebrauchsposition zu positionieren. Es kann ferner auch bei einem Analysesystem eingesetzt werden, das ein Trägerband mit mehreren Test feldern zur Aufnahme einer Körperflüssekeitsprobe und ein Gerätegehäuse umfasst, das eine Messeinrichtung zur Untersuchung einer Körperflüssigkeitsprobe und eine Fördereinrichtung enthält, um die Testfelder durch Bewegen des Trägerbandes in einer Förderrichtung nacheinander in eine Gebrauchsposition zu bringen, in der diese eine Körperfigssigkeitsprobe aufnehmen oder mit der Messeinrichtung zusammenwirken Können. Ein derartiges Analysesystem ist gekennzeichnet durch Positionsmarken, die das Trägerband aufweist, ein Speichermedium, auf dem für die Testfelder Entfernungswerte gespeichert sind, die von der Entfernung eines Testfeldes des Trägerbandes von einer ihm zugeordneten Positionsmarke abhängen, und einen Sensor zum Detektieren von Positionsmarken in einer Detektionsposition, welche die Positionsmarken bei einer von der Fördereinrichtung bewirkten Bewegung des Trägerbandes durchlaufen, und eine an den Sensor angeschlossene Steuereinheit zum Steuern der Fördereinrichtung, wobei zum Positionieren eines Testfeldes in der Gebrauchsposition die Steuereinheit die Fördereinrichtung stoppt, sobald das Trägerband um einen Positionierweg in der Förderrichtung über eine Bandposition, in der eine Positionsmarke die Detektionsposition erreicht hat, hinausbewegt wurde, wobei die Länge des Positionierwegs von der Entfernung des zu positionierenden Testfeldes von der detektierten Positionsmarke abhängt und von der Steuereinheit mittels des auf dem Speichermedium für das zu positionierende Testfeld gespeicherten Entfernungswerts bestimmt wird. Ein solches Analysesystem kann in ein erfindungsgemäßes Stechsystem integriert sein oder unabhängig davon realisiert werden.

Weitere Einzelheiten und Vorteile der Erfindung werden anhand eines Ausführungsbeispiels unter Bezugnahme auf die beigefügten Zeichnungen erläutert. Die dabei beschriebenen Merkmale können einzeln oder in Kombination zum Gegenstand von Ansprüchen gemacht werden. Es zeigen:
- Figur 1: ein Ausführungsbeispiel eines erfindungsgemäßen Trägerbandes;
- Figur 2: eine schematische Darstellung eines Ausführungsbeispiels eines Stechsystems; und
- Figur 3: ein Beispiel eines Sensorsignals, das sich beim Vorbeiführen eines Markierungssegments einer Positionsmarkierung des in Figur 1 dargestellten Trägerbandes an einem Sensor ergibt.

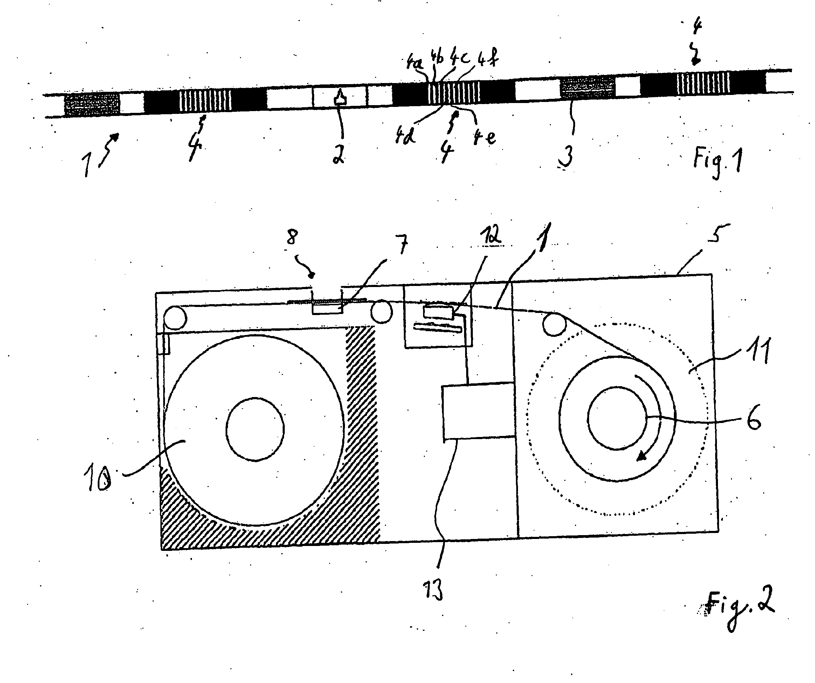

Das in Figur 1 dargestellte Trägerband 1 trägt als Funktionselemente Lanzetten 2 und Testfelder 3, die jeweils zwischen Lanzetten 2 angeordnet sind. Die Lanzetten 2 dienen dazu, eine Stichwunde zur Gewinnung einer Körperflüssigkeitsprobe zu erzeugen, die mittels eines Testfeldes 3 zum Nachweis oder zur Konzentrationsbestimmung eines Analyten untersucht werden kann. Bei dem dargestellten Ausführungsbeispiel sind die Lanzetten 2 quer zur Längsrichtung des Bandes 1 orientiert. Es ist aber auch möglich, die Lanzetten auf dem Trägerband so anzuordnen, dass ihre Spitze in Längsrichtung des Trägerbandes 1 zeigt.

Auf dem Trägerband 1 ist für jedes Funktionselement 2, 3 eine Positionsmarke 4 angebracht. Bei dem dargestellten Ausführungsbeispiel sind die Positionsmarken 4 jeweils zwischen den Funktionselementen 2, 3 angeordnet. Die Anzahl der Positionsmarken 4 kann prinzipiell von den dargestellten Verhältnissen abweichen. Beispielsweise ist es möglich, für zwei aufeinander folgende Funktionselemente 2, 3 nur eine einzige Positionsmarke 4 vorzusehen oder mehr Positionsmarken 4 als bei dem dargestellten Ausführungsbeispiel einzusetzen.

Bei dem dargestellten Ausführungsbeispiel sind die Positionsmarken 4 jeweils aus mehreren Markierungssegmenten 4a, 4b, 4c, 4d, 4e, 4f aufgebaut, die in Längsrichtung des Bandes nebeneinander angeordnet sind. Die Markierungssegmente können gemäß Figur 1 als Striche oder auch in einer anderen geometrischen Form, beispielsweise als Punkte ausgebildet sein. Durch eine Variation der Positionsmarken 4, beispielsweise der Breite des ersten Markierungssegments 4a, kann gekennzeichnet werden, ob das in Förderrichtung nächstliegende Funktionselement eine Lanzette 2 oder ein Testfeld 3 ist.

Bei der Herstellung des Trägerbandes 1 definieren die Positionsmarken 4 Sollpositionen, in denen die Funktionselemente 2, 3 angeordnet werden sollen. Bei dem dargestellten Ausführungsbeispiel sind diese Sollpositionen jeweils in der Mitte zwischen Positionsmarken 4. Beim Aufbringen der Funktionselemente 2, 3 wird aus Interesse an einer schnellen und kostengünstigen Fertigung bewusst in Kauf genommen, dass zumindest ein Teil der Funktionselemente 2, 3 mit einer unter Umständen erheblichen Abweichung von der durch die Positionsmarken 4 vorgegeben Sollposition auf dem Trägerband 1 platziert wird. Nach dem Plazieren der Funktionselemente 2, 3 wird deshalb für die Funktionselemente 2, 3 der Abstand von einer ihnen zugeordneten Positionsmarke 4 gemessen und ein daraus ermittelter Entfernungswert auf einem Speichermedium gespeichert.

Die Entfernungswerte hängen bei dem dargestellten Ausführungsbeispiel von dem Abstand der Spitze einer Lanzette 2 von einer benachbarten, bevorzugt der entgegen der Förderrichtung nächstliegenden, Positionsmarke 4 ab. Entfernungswerte für Testfelder 3 hängen von der Entfernung eines Feldrandes von einer benachbarten, bevorzugt der entgegen der Förderrichtung nächstliegenden, Positionsmarke 4 ab. Die Entfernungswerte werden auf einem Speichermedium gespeichert, beispielsweise als Barcode auf der Rückseite des Trägerbandes 1. Möglich ist es auch, auf der Rückseite des Trägerbandes 1 ein magnetisches Speichermedium anzuordnen oder das Trägerband in einer Bandkassette anzuordnen, die zum Einsetzen in ein Stechgerät bestimmt ist, und das Speichermedium an der Bandkassette anzubringen, so dass die Entfernungswerte von einem Lesegerät eines Stechgeräts, in das die Bandkassette eingesetzt ist, gelesen werden können.

Unter der Förderrichtung ist dabei die Richtung in Längsrichtung des Trägerbandes 1 zu verstehen, in das Trägerband 1 bewegt werden muss, um unbenutzte Funktionselemente 2, 3 in die Gebrauchsposition und benutzte Funktionselemente 2, 3 weg von der Gebrauchsposition zu bewegen.

Das Trägerband 1 bildet zusammen mit dem Speichermedium, auf dem die Entfernungswerte gespeichert sind, ein Lanzettenvorratssystem. Ein derartiges Lanzettenvorratssystem kann mit einem Stechgerät verwendet werden, in das ein solches Trägerband 1 eingesetzt und nach Gebrauch aller Lanzetten 2 gegen ein frisches Trägerband 1 ausgetauscht werden kann. Das in Figur 1 dargestellte Trägerband 1 kann jedoch auch in einem Gerät verwendet werden, das einen Austausch des Trägerbandes 1 nicht vorsieht und als so genanntes Einweggerät entsorgt wird, sobald alle Lanzetten 2 des Trägerbandes 1 benutzt wurden.

In Figur 2 ist schematisch ein Ausführungsbeispiel eines Stechsystems mit dem in Figur 1 dargestellten Trägerband 1 gezeigt. Das Trägerband 1 ist in einem Gerätegehäuse 5 angeordnet, das eine Fördereinrichtung 6 enthält, um die Funktionselemente 2, 3 des Trägerbands 1 durch Bewegen des Trägerbandes 1 in einer Förderrichtung nacheinander in eine Gebrauchsposition zu bringen. In dem Gerätegehäuse ist ferner ein Stechantrieb 7 angeordnet, um eine in der Gebrauchsposition positionierte Lanzetten 2 für eine Stechbewegung zu beschleunigen, so dass mit einer Stechbewegung in ein an die Gehäuseöffnung 8 angelegtes Körperteil eingestochen werden kann. Befindet sich ein Testfeld 3 in der Gebrauchsposition vor der Geräteöffnung 8, kann darauf eine durch einen Stich gewonnene Körperflüssigkeitsprobe aufgebracht werden.

Bei dem dargestellten Ausführungsbeispiel ist das Trägerband 1 ähnlich wie in einer Tonbandkassette auf zwei Rollen 10, 11 aufgewickelt. Eine dieser beiden Rollen ist durch die Fördereinrichtung 6 angetrieben, so dass das Band mittels der Fördereinrichtung 6 von einer ersten Rolle 10 abgewickelt und auf eine angetriebene zweite Rolle 11 aufgewickelt wird und auf diese Weise in Förderrichtung bewegt werden kann. Anstelle der nicht-angetriebenen Rolle 10 kann beispielsweise auch ein Stapel genutzt werden, zu dem unbenutzte Abschnitte des Trägerbandes 1 gefaltet sind.

Das Trägerband 1 läuft an einem Sensor 12 vorbei. Der Sensor 12 detektiert Positionsmarken 4 in einer Detektionsposition, welche die Positionsmarken 4 bei einer von der Fördereinrichtung 6 bewirkten Bewegung des Trägerbandes 1 durchlaufen. Bei dem dargestellten Ausführungsbeispiel befindet sich eine Positionsmarke 4 in der Detektionsposition, wenn sie sich unmittelbar vor dem Sensor 12 befindet.

Bei dem Sensor 12 handelt es sich bevorzugt um einen optischen Sensor. Auf diese Weise kann nämlich der Sensor 12 auch als Messeinrichtung zum photometrischen Untersuchen einer Verfärbung eines Testfelds 3, die durch einen in einer Körperflüssigkeitsprobe enthaltenen Analyten bewirkt wird, genutzt werden. Testfelder zum photometrischen Nachweis oder zur photometrischen Konzentrationsbestimmung von Analyten und dazu passende Messeinrichtungen sind bei handelsüblichen Geräten zur Blutzuckerüberwachung vorhanden, so dass nähere Erläuterungen nicht erforderlich sind. Wird die Messeinrichtung zur photometrischen Untersuchung einer Verfärbung eines Testfeldes 3 zugleich als Sensor 12 zum Detektieren von Positionsmarken 4 genutzt, lassen sich Kosten einsparen und der Aufbau des Stechsystems vereinfachen. Selbstverständlich ist es aber auch möglich, Positionsmarken 4 zu verwenden, die nicht auf optischem Wege detektiert werden, beispielsweise magnetische Positionsmarken und entsprechende magnetische Sensoren.

Bei Verwendung eines optischen Sensors 12 ist es besonders günstig, Positionsmarken 4 in Reflektion zu detektieren. Dafür ist es günstig, die Positionsmarken aus Material mit einer möglichst hohen Reflektivität zu bilden, beispielsweise weiße Farbe oder Metallschichten, die zu Bereichen geringer Reflektivität, insbesondere schwarzen Bereichen, einen hohen Kontrast liefern. Wird ein optischer Sensor verwendet, der Fluoreszenz misst, können auch fluoreszierende Pigmente oder Fluoreszenzfarbstoffe eingesetzt werden. Die Positionsmarken 4 können auf das Trägerband 1 aufgedruckt sein. An sich sind alle Druckverfahren möglich. Bevorzugt sind Siebdruck, insbesondere Zylindersiebdruck, Laserdruck oder Tintenstrahldruck. Metallische Striche können durch Transferdruck oder beispielsweise durch partielle Ablation einer vorher aufgedampften Metallschicht, beispielsweise mit einem Laser, gebildet werden.

Der Sensor 12 ist an eine Steuereinheit 13 angeschlossen, die zum Positionieren eines Funktionselementes 2, 3 in der Gebrauchsposition die Fördereinrichtung 6 stoppt, sobald das Trägerband 1 um eine Strecke in der Förderrichtung über eine Bandposition, in der eine Positionsmarke 4 die Detektionsposition erreicht hat, hinausbewegt wurde, wobei die Länge dieser Strecke von der Entfernung des zu positionierenden Funktionselement 2, 3 von der delektierten Positionsmarke 4 abhängt und von der Steuereinheit 13 mittels des auf dem Speichermedium für das zu positionierende Funktionselement 2, 3 gespeicherten Entfernungswertes bestimmt wird. Die Steuereinheit 13 kann beispielsweise ein ASIC oder ein Mikroprozessor sein.

Wie bereits erwähnt sind die Positionsmarken 4 aus mehreren Markierungssegmenten 4a, 4b, 4c, 4d, 4e, 4f, aufgebaut, die in Längsrichtung des Trägerbandes 1 nebeneinander angeordnet sind. In Figur 1 sind beispielhaft nur die ersten 6 Markierungssegmente mit Bezugszeichen versehen. Die genaue Anzahl der Markierungssegmente kann weitgehend frei gewählt werden. Bei dem dargestellten Ausführungsbeispiel haben mehrere Markierungssegmente der Positionsmarken 4 eine übereinstimmende Ausdehnung in Längsrichtung des Bandes. Die in Figur 1 gezeigten Positionsmarken 4 haben als Markierungssegmente aufeinander folgende helle und dunkle Bereiche, die jeweils gleiche Ausdehnung in Längsrichtung des Bandes haben.

Der Sensor 12 umfasst eine Lichtquelle, die im Betrieb Licht aussendet, das in einem Messfleck auf dem Trägerband 1 fokussiert wird. Der Messfleck hat dabei in Förderrichtung des Trägerbandes 1 eine Ausdehnung, die höchstens 30 %, vorzugsweise höchstens 20 %, von der sich in Förderrichtung erstreckenden Ausdehnung eines dieser Markierungssegmente 4c, 4d, 4e, 4f abweicht. Bei dem dargestellten Ausführungsbeispiel stimmt die Ausdehnung des Messflecks in Förderrichtung mit der entsprechenden Ausdehnung dieser Markierungssegmente überein. Diese Maßnahme hat den Vorteil, dass sich eine Positionsauflösung erreichen lässt, die nur einen Bruchteil der Ausdehnung eines Markierungssegments in Förderrichtung umfasst. Die Auflösung wird im wesentlichen nur durch die Präzision und Reproduzierbarkeit der Breite der Markierungssegmente und die Ungenauigkeit des Messfleckdurchmessers begrenzt.

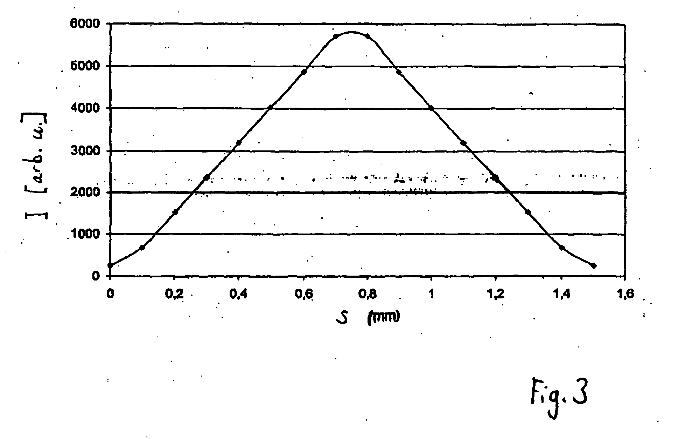

Figur 3 zeigt ein Ausführungsbeispiel eines Sensorsignals, das sich beim Vorbeiführen eines Markierungssegments an dem Sensor 12 ergibt. Dabei ist die Signalintensität in willkürlichen Einheiten (arb. u.) über der von dem Trägerband 1 zurückgelegten Wegstrecke s im Millimetern aufgetragen. Eine maximale Signalintensität ergibt sich dabei genau dann, wenn ein Markierungssegment vollständig von dem Messfleck bedeckt wird, da sich dann maximale Reflektion ergibt. Bei einer nur partiellen Bedeckung eines Markierungssegments durch den Messfleck ergibt sich ein entsprechend reduziertes Signal. Das Sensorsignal wird also analog ausgewertet. Aus dem Extremum des Sensorsignals wird darauf geschlossen, dass ein Markierungssegment vollständig von dem Messfleck bedeckt ist.

Um ein Funktionselement 2, 3 in der Gebrauchsposition zu positionieren, wird zunächst das Band 1 in eine Bandposition befördert, in der sich die Positionsmarke 4 in der Dektionsposition befindet und der Sensor 12 ein entsprechendes Detektionssignal liefert. Von dieser Bandposition aus muss das Band 1 dann noch um einen Positionierweg in Förderrichtung bewegt werden, dessen Länge von dem für das zu positionierende Funktionselement 2, 3 gespeicherten Entfernungswert abhängt. Die Detektionsposition kann dabei beispielsweise so definiert sein, dass das Sensorsignal ein erstes Extremum erreicht. Bei dem dargestellten Ausführungsbeispiel ist dies der Fall, wenn der Messfleck des Sensors 12 das erste Markierungssegment 4a vollständig bedeckt. Möglich ist es aber auch, die Detektionsposition, welche in Kombination mit dem Entfernungswert den noch erforderlichen Positionierweg festlegt, dahingehend zu definieren, dass die Detektionsposition beispielsweise erst bei dem zweiten oder dritten Extremum des Sensorsignals erreicht ist.

Durch die Verwendung von Positionsmarken 4, die jeweils aus mehreren Markierungssegmenten aufgebaut sind, lässt sich erreichen, dass der Sensor 12 ein Sensorsignal liefert, das sich ändert, während das Trägerband 1 um den Positionierweg über eine Bandposition, in der eine Positionsmarke 4 die Detektionsposition erreicht hat, hinausbewegt wird. Bei dem dargestellten Ausführungsbeispiel ist die Ausdehnung der Positionsmarken 4 in Förderrichtung so groß gewählt, dass diese größer als die Positionierwege sind, um die das Trägerband 1 noch bewegt werden muss, wenn die Detektionsposition erreicht ist, um ein Funktionselement 2, 3 in der Gebrauchsposition zu positionieren. Auf diese Weise kann durch Auswertung des sich ändernden Sensorsignals ermittelt werden, wann das Trägerband 1 um den Positionierweg bewegt wurde und folglich die Fördereinrichtung 6 zu stoppen ist. Zwischen den Positionsmarken 4 erstreckt sich dabei ein Bandabschnitt, der die Funktionselemente 2, 3 trägt und frei von Positionsmarken ist.

Um ein Funktionselement 2, 3 in der Gebrauchsposition zu positionieren, wird bei dem dargestellten Ausführungsbeispiel die Fördergeschwindigkeit reduziert, sobald ein erstes Markierungssegment 4a von dem Sensor 12 erfasst wird. Auf diese Weise kann bei einer reduzierten Geschwindigkeit mit einer höheren Präzision ermittelt werden, wann die Positionsmarke die Detektionsposition erreicht hat, also ein vorgegebenes Markierungssegment vollständig von dem Sensor 12 erfasst wird. Prinzipiell kann hierfür das zweite Markierungssegment 4b verwendet werden. Es hat sich jedoch gezeigt, dass sich eine größere Genauigkeit erzielen lässt, wenn zunächst mit dem zweiten Markierungssegment eine Kalibrierung des Sensors 12 vorgenommen wird. Bei dem dargestellten Ausführungsbeispiel ist die Detektionsposition von einer Positionsmarke 4 genau dann erreicht, wenn deren drittes Markierungssegment 4c von dem Sensor 12 erfasst wird.

Die Funktionselemente, insbesondere die Lanzetten 2, sind auf dem Trägerband 1 zwischen den Positionsmarken 4 mit einer Positionierungsungenauigkeit angeordnet. Die Positionsmarken 4 weichen dabei von einer beim Fertigungsprozess angestrebten Sollposition um eine Distanz ab, die kleiner als die Ausdehnung einer Positionsmarken 4, also der Markierungssegmente einer Positionsmarke 4 insgesamt ist. Befindet sich eine Positionsmarke 4 in der Detektionsposition, so ist die zugeordnete Lanzette 2 von der Gebrauchsposition weniger weit entfernt als sich die Positionsmarke 4 mit noch nicht von dem Sensor 12 erfassten Segmente 4e, 4f in Förderrichtung erstreckt. Auf diese Weise kann aus dem Entfernungswert präzise ermittelt werden, wie viele Markierungssegmente der aktuellen Positionsmarke 4 an dem Sensor 12 vorbeigeführt werden müssen, bis die Lanzette 2 die Gebrauchsposition erreicht hat. Da mit den anhand von Figur 3 erläuterten Verfahren der Signalauswertung eine Positionienrngsgenauigkeit erreicht werden kann, die besser als die Ausdehnung der einzelnen Markierungssegmente in Förderrichtung ist, kann das Trägerband genau dann gestoppt werden, wenn die Lanzette 2 optimal positioniert ist.

Neben den Entfernüngswerten können auf dem Speichermedium auch Informationen über fehlerhafte Funktionselemente 2, 3 oder Funktionselemente, die außerhalb eines akzeptablen Toleranzbereiches auf dem Trägerband 1 positioniert sind, gespeichert werden. Derartige Funktionselemente 2, 3 können beim Betrieb des Stechsystems dann einfach übergangen werden.

### Bezugszahlenliste

- 1: Trägerband
- 2: Lanzette
- 3: Testfeld.
- 4: Positionsmarke
- 4a: Markierungssegmente
- 4b: Markierungssegmente
- 4c: Markierungssegmente
- 4d: Markierungssegmente
- 4e: Markierungssegmente
- 4f: Markierungssegmente
- 5: Gerätegehäuse
- 6: Fördereinrichtung
- 7: Stechantrieb
- 8: Gehäuseöffnung
- 9:
- 10: Wickelspule
- 11: Wickelspule
- 12: Sensor
- 13: Steuereinheit

## Patentansprüche

1. Stechsystem mit
einem Trägerband (1), das mehrere Lanzetten (2) trägt und Positionsmarken (4) aufweist, und
einem Gerätegehäuse (5), das eine Fördereinrichtung (6), um die Lanzetten (2) durch Bewegen des Trägerbandes (1) in einer Förderrichtung nacheinander in eine Gebrauchsposition zu bringen, und einen Stechantrieb (7), um eine in der Gebrauchsposition positionierte Lanzette (2) für eine Stechbewegung zu beschleunigen, enthält und eine Gehäuseöffnung (8) zum Anlegen eines Körperteils, in das mit einer Stechbewegung einer Lanzette (2) eingestochen werden soll, aufweist,
einem Sensor (12) zum Detektieren von Positionsmarken (4) in einer Detektionsposition, welche die Positionsmarken (4) bei einer von der Fördereinrichtung (6) bewirkten Bewegung des Trägerbandes (1) durchlaufen, und
eine an den Sensor (12) angeschlossene Steuereinheit (13) zum Steuern der Fördereinrichtung (6), wobei die Steuereinheit (13) zum Positionieren einer Lanzette (2) in der Gebrauchsposition die Fördereinrichtung (6) stoppt, sobald das Trägerband (1) um einen Positionierweg beginnend bei eine Bandposition, in der eine Positionsmarke (4) die Detektionsposition erreicht hat, bewegt wurde,
**gekennzeichnet durch**
ein Speichermedium, auf dem für die Lanzetten (2) Entfernungswerte gespeichert sind, die von der Entfernung einer Lanzette (2) des Trägerbandes (1) von einer ihr zugeordneten Positionsmarke (4) abhängen,
wobei die Länge des Positionierwegs von der Entfernung der zu positionierenden Lanzette (2) von der detektierten Positionsmarke (4) abhängt und von der Steuereinheit (13) mittels des auf dem Speichermedium für die zu positionierende Lanzette (2) gespeicherten Entfernungswerts bestimmt wird.

2. Stechsystem nach Anspruch 1, **dadurch gekennzeichnet, dass** das Trägerband (1) von der Fördereinrichtung (6) stets nur in der Förderrichtung bewegt wird.

3. Stechsystem nach einem der vorstehenden Ansprüche, **dadurch gekennzeichnet, dass** der Sensor (12) ein Sensorsignal liefert, das sich ändert, während das Trägerband (1) um den Positionierweg über eine Bandposition, in der eine Positionsmarke (4) die Detektionsposition erreicht hat, hinausbewegt wird, wobei die Steuereinheit (13) durch Auswertung des sich ändernden Sensorsignals ermittelt, wann das Trägerband (1) um den Positionierweg bewegt und folglich die Fördereinrichtung (6) zu stoppen ist.

4. Stechsystem nach einem der vorstehenden Ansprüche, **dadurch gekennzeichnet, dass** die Positionsmarken (4) jeweils aus mehreren Markierungssegmenten (4a, 4b, 4c, 4d, 4e, 4f) aufgebaut sind, die in Längsrichtung des Trägerbandes (1) nebeneinander angeordnet sind.

5. Stechsystem nach Anspruch 4, **dadurch gekennzeichnet, dass** die Markierungssegmente (4a, 4b, 4c, 4d, 4e, 4f) in einem Abstand angeordnet sind, der ihrer Breite entspricht.

6. Stechsystem nach einem der vorstehenden Ansprüche, **dadurch gekennzeichnet, dass** der Sensor (12) ein optischer Sensor ist.

7. Stechsystem nach Anspruch 6, **dadurch gekennzeichnet, dass** der Sensor (12) eine Lichtquelle umfasst, die im Betrieb Licht aussendet, das in einem Messfleck auf dem Trägerband (1) fokussiert wird, wobei der Messfleck in Förderrichtung des Trägerbandes (1) eine Ausdehnung hat, die höchstens 30 %, vorzugsweise höchstens 20 %, von der sich in Förderrichtung erstreckenden Ausdehnung eines Markierungssegments (4c, 4d, 4e, 4f) abweicht, vorzugsweise mit dieser übereinstimmt.

8. Stechsystem nach einem der vorstehenden Ansprüche, **dadurch gekennzeichnet, dass** auf dem Trägerband (1) zwischen den Lanzetten (2) Testfelder (3) zur Untersuchung einer aus einer Stichwunde gewonnenen Körperflüssigkeitsprobe angeordnet sind.

9. Stechsystem nach einem der vorstehenden Ansprüche, **dadurch gekennzeichnet, dass** der Sensor (12) eine Messeinrichtung zum photometrischen Untersuchung einer Verfärbung eines Testfelds (3) ist, die durch einen in einer Körperflüssigkeitsprobe enthaltenen Analyten bewirkt wird.

10. Stechsystem nach einem der vorstehenden Ansprüche, **dadurch gekennzeichnet, dass** auf dem Speichermedium eine Information über Lanzetten (2), die außerhalb eines Toleranzbereichs auf dem Trägerband (1) positioniert oder fehlerhaft sind, gespeichert ist, wobei diese Information von der Steuereinheit (13) ausgewertet wird, so dass Lanzetten (2), die außerhalb eines Toleranzbereichs auf dem Trägerband (1) positioniert oder fehlerhaft sind, beim Betrieb übergangen und stattdessen jeweils die in Förderrichtung nächstliegende Lanzette (2) in der Gebrauchsposition positioniert wird.

11. Lanzettenvorratssystem für ein Stechsystem mit einem Trägerband (1), das mehrere Lanzetten (2) trägt und Positionsmarken (4) aufweist, **gekennzeichnet durch** ein Speichermedium, auf dem für die Lanzetten (2) Entfernungswerte gespeichert sind, die jeweils von der Entfernung einer Lanzette (2) des Trägerbandes (1) von einer ihr zugeordneten Positionsmarke (4) abhängen.

12. Lanzettenvorratssystem nach Anspruch 11, **dadurch gekennzeichnet, dass** auf dem Trägerband (1) zwischen den Lanzetten.(2) Testfelder (3) zur Untersuchung einer aus einer Stichwunde gewonnenen Körperflüssigkeitsprobe angeordnet sind

13. Verfahren zur Herstellung eines Lanzettenvorratssystems mit den folgenden Schritten: Bereitstellen eines Trägerbandes (1), Versehen des Trägerbandes (1) mit Positionsmarken (4), Anbringen der Lanzetten (2) auf dem Trägerband (1), Messen der Entfernungen von Lanzetten (2) zu einer ihnen zugeordneten Positionsmarke (4), Speichern von Entfernungswerten, aus denen diese Entfernungen ermittelbar sind, auf einem Speichermedium.

14. Verfahren zum Positionieren von auf einem Trägerband (1) angeordneten Lanzetten (2) oder Testfeldem (3) in einer Gebrauchsposition durch Bewegen des Trägerbandes (1) in Längsrichtung, wobei auf dem Trägerband (1) angebrachte Positionsmarken (4) mittels eines Sensors (12) detektiert werden, **dadurch gekennzeichnet, dass** eine Bewegung des Trägerbandes (1) um einen Positionierweg bewirkt wird, dessen Länge von einem gespeicherten Entfernungswert abhängt, der von der Entfernung der zu positionierenden Lanzette (2) oder des zu positionierenden Testfelds (3) von der detektierten Positionsmarke (4) abhängt.

## Claims

1. Puncturing system comprising
a carrier tape (1) that carries multiple lancets (2) and position marks (4), and
a device housing (5) that contains a conveying facility (6) for positioning the lancets (2) consecutively in a usage position by moving the carrier tape (1) in a conveying direction, and a puncturing drive (7) for accelerating a lancet (2) that is positioned in the usage position for a puncturing motion, and has a housing opening (8) for touching against a body part, in which a puncture is to be made by a puncturing motion of a lancet (2),
a sensor (12) for detecting position marks (4) in a detection position through which the position marks (4) proceed upon a motion of the carrier tape (1) that is effected by the conveying facility (6); and
a control unit (13) that is connected to the sensor (12) for controlling the conveying facility (6), whereby, in order to position a lancet (2) in the usage position, the control unit (13) stops the conveying facility (6) as soon as the carrier tape (1) has been moved by a positioning distance starting from a tape position, in which a position mark (4) has reached the detection position,
**characterized in that**
a storage medium on which distance values for the lancets (2) are stored, said distance values depending on the distance between a lancet (2) of the carrier tape (1) and a position mark (4) that is allocated to it;
whereby the length of the positioning distance depends on the distance between the lancet (2) to be positioned and the detected position mark (4) and is determined by the control unit (13) by means of the distance value that is stored on the storage medium for the lancet (2) to be positioned.

2. Puncturing system according to claim 1, **characterized in that** the carrier tape (1) is always moved by the conveying facility (6) in conveying direction only.

3. Puncturing system according to any one of the preceding claims, **characterized in that** the sensor (12) produces a sensor signal that changes while the carrier tape (1) is moved by the positioning distance past a tape position, in which a position mark (4) has reached the detection position, whereby the control unit (13) determines by analysis of the changing sensor signal when the carrier tape (1) has been moved by the positioning distance and, accordingly, the conveying facility (6) is to be stopped.

4. Puncturing system according to any one of the preceding claims, **characterized in that** the position marks (4) each are made up of multiple marking segments (4a, 4b, 4c, 4d, 4e, 4f) that are arranged adjacent to each other in longitudinal direction of the carrier tape (1).

5. Puncturing system according to claim 4, **characterized in that** the marking segments (4a, 4b, 4c, 4d, 4e, 4f) are arranged at a distance that corresponds to their width.

6. Puncturing system according to any one of the preceding claims, **characterized in that** the sensor (12) is an optical sensor.

7. Puncturing system according to claim 6, **characterized in that** the sensor (12) comprises a light source, which, in operation, emits light that is focussed into a measuring spot on the carrier tape (1), whereby the extension of the measuring spot in conveying direction of the carrier tape (1) deviates at most by 30%, preferably at most by 20%, from the extension in conveying direction of a marking segment (4c, 4d, 4e, 4f), preferably coincides with it.

8. Puncturing system according to any one of the preceding claims, **characterized in that** test fields (3) for testing a sample of body fluid that is obtained from a puncturing wound are arranged between the lancets (2) on the carrier tape (1).

9. Puncturing system according to any one of the preceding claims, **characterized in that** the sensor (12) is a measuring facility for photometric measurement of a color change of a test field (3) that is effected by an analyte that is contained in a sample of body fluid.

10. Puncturing system according to any one of the preceding claims, **characterized in that** information concerning lancets (2) that are positioned on the carrier tape (1) outside an acceptable tolerance range or are flawed is stored on the storage medium, whereby this information is analyzed by the control unit (13) such that lancets (2) that are positioned on the carrier tape (1) outside an acceptable tolerance range or are flawed are passed over in operation and, instead, the next closest lancet (2) in conveying direction is positioned in the usage position.

11. Lancet reservoir system for a puncturing system comprising a carrier tape (1) that carries multiple lancets (2) and has position marks (4), **characterized by** a storage medium on which distance values for the lancets (2) are stored, which each depend on the distance between a lancet (2) of the carrier tape (1) and a position mark (4) allocated to it.

12. Lancet reservoir system according to claim 11, **characterized in that** test fields (3) for testing a sample of body fluid that is obtained from a puncturing wound are arranged between the lancets (2) on the carrier tape (1).

13. Method for the manufacture of a lancet reservoir system including the following steps: providing a carrier tape (1), placing position marks (4) on said carrier tape (1), placing the lancets (2) on said carrier tape (1), measuring the distances between lancets (2) and a position mark (4) that is allocated to them, storing of distance values from which these distances can be determined on a storage medium.

14. Method for the positioning of lancets (2) or test fields (3) that are arranged on a carrier tape (1) in a usage position by moving the carrier tape (1) in longitudinal direction, wherein position marks (4) that are placed on the carrier tape (1) are detected by means of a sensor (12), **characterized in that** a motion of the carrier tape (1) by a positioning distance is effected, whereby the length of said positioning distance depends on a stored distance value that depends on the distance between the lancet (2) or the test field (3) to be positioned and the detected position mark (4).

## Revendications

1. Système de piquage comprenant
une bande support (1), qui porte plusieurs lancettes (2) et présente des repères de position (4), et
un carter d'appareil (5), qui contient un système de transport (6) pour amener les lancettes (2) successivement dans une position d'utilisation par déplacement de la bande support (1) dans une direction de transport, et un entraînement de piquage (7) pour accélérer une lancette (2) positionnée dans la position d'utilisation pour un mouvement de piquage et présente une ouverture de carter (8) pour l'application d'une partie du corps, dans laquelle on doit piquer avec un mouvement de piquage d'une lancette (2),
un capteur (12) pour la détection de repères de position (4) dans une position de détection, par laquelle passent les repères de position (4) lors d'un déplacement, causé par le système de transport (6), de la bande support (1), et
une unité de commande (13) raccordée au capteur (12) pour la commande du système de transport (6), l'unité de commande (13) arrêtant le système de transport (6) pour le positionnement d'une lancette (2) dans la position d'utilisation dès que la bande support (1) a été déplacée d'une course de positionnement en commençant sur une position de bande dans laquelle un repère de position (4) a atteint la position de détection,
**caractérisé par**
un support de stockage sur lequel des valeurs de distance sont mémorisées pour les lancettes (2), lesquelles valeurs dépendent de la distance d'une lancette (2) de la bande support (1) à un repère de position (4) attribué à la lancette,
la longueur de la course de position dépendant de la distance de la lancette (2) à positionner au repère de position (4) à détecter et étant déterminées par l'unité de commande (13) au moyen de la valeur de distance mémorisée sur le support de stockage pour la lancette (2) à positionner.

2. Système de piquage selon la revendication 1, **caractérisé en ce que** la bande support (1) est déplacée par le système de transport (6) toujours uniquement dans la direction de transport.

3. Système de piquage selon l'une quelconque des revendications précédentes, **caractérisé en ce que** le capteur (12) fournit un signal de capteur qui varie, lorsque la bande support (1) est déplacée de la course de positionnement au-delà d'une position de bande dans laquelle un repère de position (4) a atteint la position de détection, l'unité de commande (13) déterminant par l'analyse du signal de capteur variable quand la bande support (1) est déplacée de la course de positionnement et en conséquence le système de transport (6) doit être arrêté.

4. Système de piquage selon l'une quelconque des revendications précédentes, **caractérisé en ce que** les repères de position (4) sont constitués chacun de plusieurs segments de marquage (4a, 4b, 4c, 4d, 4e, 4f), qui sont disposés dans le sens longitudinal de la bande support (1) les uns à côté des autres.

5. Système de piquage selon la revendication 4, **caractérisé en ce que** les segments de marquage (4a, 4b, 4c, 4d, 4e, 4f) sont disposés à une distance qui correspond à leur largeur.

6. Système de piquage selon l'une quelconque des revendications précédentes, **caractérisé en ce que** le capteur (12) est un capteur optique.

7. Système de piquage selon la revendication 6, **caractérisé en ce que** le capteur (12) comprend une source lumineuse qui émet en service de la lumière qui est concentrée dans un spot de mesure sur la bande support (1), le spot de mesure ayant dans la direction de transport de la bande support (1) une étendue qui diffère d'au maximum 30%, de préférence d'au maximum 20%, de l'étendue, dans la direction de transport, d'un segment de marquage (4c, 4d, 4e, 4f), et de préférence coïncide avec celle-ci.

8. Système de piquage selon l'une quelconque des revendications précédentes, **caractérisé en ce que** des zones test (3) sont disposées sur la bande support (1) entre les lancettes (2) pour l'examen d'un échantillon de liquide corporel obtenu à partir d'une blessure de piquage.

9. Système de piquage selon l'une quelconque des revendications précédentes, **caractérisé en ce que** le capteur (12) est un dispositif de mesure pour l'examen photométrique d'une coloration d'une zone test (3), qui est effectuée par un analyte contenu dans un échantillon de liquide corporel.

10. Système de piquage selon l'une quelconque des revendications précédentes, **caractérisé en ce qu'**une information sur des lancettes (2), qui sont positionnées à l'extérieur d'une zone de tolérance sur la bande support (1) ou sont défectueuses, est mémorisée sur le support de stockage, cette information étant analysée par l'unité de commande (13), de sorte que des lancettes (2), qui sont positionnées à l'extérieur d'une zone de tolérance sur la bande support (1) ou sont défectueuses, ne sont pas prises en compte lors du service et, à la place, à chaque fois la lancette (2) la plus proche dans la direction de transport est positionnée dans la position d'utilisation.

11. Système de réserve de lancettes (2) pour un système de piquage comprenant une bande support (1), qui porte plusieurs lancettes (2) et présente des repères de position (4), **caractérisé par** un support de stockage sur lequel sont mémorisées pour les lancettes (2) des valeurs de distance qui dépendent à chaque fois de la distance d'une lancette (2) de la bande support (1) à un repère de position (4) attribué à la lancette (2).

12. Système de réserve de lancettes selon la revendication 11, **caractérisé en ce que** des zones test (3) sont disposées sur la bande support (1) entre les lancettes (2) pour l'examen d'un échantillon de liquide corporel obtenu à partir d'une blessure de piquage.

13. Procédé pour la fabrication d'un système de réserve de lancettes comprenant les étapes suivantes : mise à disposition d'une bande support (1), équipement de la bande support (1) avec des repères de position (4), placement des lancettes (2) sur la bande support (1), mesure des distances de lancettes (2) à un repère de position (4) attribué à ces lancettes, stockage de valeurs de distance à partir desquelles ces distances peuvent être déterminées, sur un support de stockage.

14. Procédé pour le positionnement de lancettes (2) ou zones test (3) disposées sur une bande support (1) dans une position d'utilisation par déplacement de la bande support (1) dans la direction longitudinale, des repères de position (4) placés sur la bande support (1) étant détectés au moyen d'un capteur (12), **caractérisé en ce qu'**un déplacement de la bande support (1) d'une course de position est occasionné, course dont la longueur dépend d'une valeur de distance mémorisée qui dépend elle-même de la distance de la lancette (2) à positionner ou de la zone test (3) à positionner au repère de position (4) détecté.
